# EUROPEAN PATENT APPLICATION

(11) **EP 2 283 838 A2**
(43) Date of publication of application: **16.02.2011**
(21) Application number: 10179837.9
(22) Date of filing: 29.09.2003
(51) Int. Cl.: A61K 31/202, A61K 31/232, A61K 31/60, A61P 9/10, A61P 29/00

(54) **Docosahexaenoic acid for treating subclinical inflammation**

(30) Priority: 27.09.2002 US 413857 P
(62) Divisional of application: 03752623.3
(71) Applicant: MARTEK BIOSCIENCES CORPORATION, Columbia, MD 21045 (US)
(72) Inventor: Arterburn, Linda, M, Ellicott City, MD 21043 (US); Hoffman, James, P, Pennsylvania, PA 19422 (US); Oken, Harry, A, 21044, Columbia (CO); Van Elswyk, Mary, 80503, Longmont (CO)
(74) Representative: Wallis, Naomi Rachel

(57) **Abstract**

This invention is directed to methods and compositions which impede the development and progression of diseases associated with subclinical inflammation. Subclinical inflammation is commonly associated with atherosclerotic cardiovascular disease, coronary disease or cerebrovascular disease. The methods and compositions of the invention are also particularly suited to providing therapy for subclinical inflammation in diabetic and prediabetic patients. Methods of the invention comprise administration of DHA alone and in combination with antiplatelet drugs.

## Description

### CROSS-REFERENCED APPLICATION

This application claims priority to U.S. Provisional application No. 60/413,857 filed on September 27, 2002, which is hereby incorporated by reference in its entirety.

### BACKGROUND

### Field of the Intention

This invention is directed to methods and compositions which impede the development and progression of diseases associated with subclinical inflammation. Subclinical inflammation is commonly associated with atherosclerotic cardiovascular disease, coronary disease or cerebrovascular disease. The methods and compositions of the invention are also particularly suited to providing therapy for subclinical inflammation in diabetic and prediabetic patients.

### Review of Related Art

Chronic non-acute systemic inflammation (subclinical inflammation) is an underlying cause of many seemingly unrelated diseases associated with aging. As humans grow older, systemic inflammation can inflict devastating degenerative effects throughout the body. Chronic inflammation has been associated with a diverse disease set that includes atherosclerosis, cancer, heart valve dysfunction, obesity, diabetes, congestive heart failure, digestive system diseases, and Alzheimer's disease. Numerous inflammatory markers exist for example, C reactive protein (CRP), various cytokines and interleukins (*e.g*. IL-1 through BL-17), TNF-alpha, e-selectin, p-selectin, sICAM, integrins, ICAM-1, ICAM-3, BL-CAM, LFA-2, VCAM, NCAM, PECAM, white blood cell count and LTB4. For instance, the inflammatory marker, C-reactive protein (CRP), is often elevated in blood tests indicating the presence of an underlying inflammatory disorder. Various methods are available for determining the levels of these inflammatory markers as an indication of the degree of inflammation (*i.e*. acute or chronic) such as enzyme-linked immuno assays (ELISA), flow cytometry and automated clinical analyzer assays.

Atherosclerosis is a degenerative disease of the arteries resulting in plaques consisting of necrotic cells, lipids, and cholesterol crystals. These plaques can result in symptoms by causing a stenosis, embolizing, and thrombosing. Atherosclerosis is a diffuse process with a predilection for certain arteries.

Stroke from any cause represents the third leading cause of death in the United States. Half a million new strokes occur each year in the United States, resulting in approximately 150,000 deaths. Stroke is the leading cause of serious long-term disability in the United States. Direct and indirect cost of stroke in the United States in 1997 was estimated at $40 billion. incidence of new stroke is approximately 160 cases per 100,000 population per year. The incidence and mortality rate of stroke have reached a plateau over the past 10 years. The risk of stroke increases with age, hypertension, the presence of a carotid bruit, diabetes, smoking, atrial fibrillation, obesity, hyperlipidemia, and elevated homocysteine.

Advances in the vascular biology of atherosclerosis indicate that inflammation plays a central role in the initiation and progression of atherothrombosis. Moreover, several systemic markers of inflammation provide important prognostic information independently of plasma lipid parameters in healthy individuals. Of these inflammatory biomarkers, CRP has been the best studied to date. Typically, CRP levels below about 1 mg/L are considered healthy. CRP levels between about 1 mg/L and about 3 mg/L indicate an increased cardiovascular risk. CRP levels between about 3 mg/L and about 10 mg/L indicate a state of chronic inflammation and increased risk for associated disorders. A CRP level above about 10 mg/L typically indicates some form of acute or clinical inflammation and is not associated with subclinical inflammation. For additional information regarding CRP levels and assessing an individuals status see U.S. patent 6,040,147.

Multiple large-scale epidemiological studies demonstrate the utility of CRP as a powerful predictor of cardiovascular events in primary prevention settings, among both men and women. Elevated levels of CRP have been associated with two- to four-fold increases in risk of first cardiovascular events in several different populations. In some studies, the predictive value of CRP has been as large as that associated with the total cholesterol to high-density lipoprotein (HDL) cholesterol ratio. Further, the addition of CRP to lipid screening appears to add to the predictive value of lipid parameters alone.

As a marker, CRP indicates an increased risk for destabilized atherosclerotic plaque, abnormal arterial clotting and for determining who is likely to suffer a heart attack. When arterial plaque becomes destabilized, it can burst open and block the flow of blood through a coronary artery, resulting in an acute heart attack. One study indicated that people with high levels of C-reactive protein were almost three times as likely to die from a heart attack (Ridker et al. 1997; New England Journal of Medicine). The American Heart Association and Centers for Disease Control & Prevention (CDC) recently endorsed the C-reactive protein test to screen for coronary-artery inflammation to identify those at risk for heart attack.

Recent studies have shown that elevated levels of inflammatory markers interleukin 6 (IL-6) and C-reactive protein (CRP) are associated with increased risk of developing Type II Diabetes Mellitus (T2DM) (Pradhan, et al., 2001, JAMA, 286:327-334). In a subsequent study, inflammatory parameters (leukocyte count, CRP and fibrinogen level) were found to be significantly correlated with insulin resistance, but not insulin secretion (Temelkova-Kurktschiev, et al., 2002, Metabolism, 51:743-749). This has lead to a hypothesis that subclinical inflammation is linked to the development of T2DM. Indeed, another study showed the mean natural logarithm of sensitive CRP was 1.05 among those who developed diabetes versus 0.53 for the remainder of subjects, indicating its strong predictive value (p < 0.0001) and that individuals with a CRP level greater than 4.18 mg/L had more than three times the risk of diabetes compared with those with CRP levels 0.66 mg/L or lower. (Diabetes 2002;51:1596-1600)

Insulin resistance (defined as the state of resistance to insulin-mediated glucose disposal and resulting compensatory hyperinsulinemia) is a characteristic off T2DM that often precedes development of the disease. Any intervention that can safely prevent or delay the onset of T2DM is of particular interest for a variety of medical and economic reasons. It is estimated that 16 million Americans are prediabetic and that 11% per year of those pre-diabetics convert to T2DM. The morbidity of T2DM (manifested by microvascular disease leading to diabetic glomerulosclerosis and end-stage renal disease, retinopathy causing blindness, and neuropathy and macrovascular disease causing accelerated atherosclerosis leading to coronary and cerebrovascular diseases such as heart attack, peripheral vascular disease and stroke) is both medically and fiscally devastating for patients. Lost productivity, high cost of medical care and mortality have a major economic impact in the workplace. Current pharmacological therapies of T2DM are increasingly reported to have characteristic side effects and resulting morbidity, such as lactic acidosis (50% fatal) and long-term 2.5-fold increase in cardiovascular (CV) mortality.

Studies on the effects of polyunsaturated fatty acids on glucose control in diabetic and prediabetic patients have to this point been inconclusive. Fish oil is a source of ω-3 polyunsaturated fatty acids including both eicosapentaenoic acid (EPA, C20:5) and docosahexaenoic acid (DHA, C22:6). Fasching, et al., (1991, Diabetes 40(5):583-589) disclosed that fish oil did not impact fasting concentrations of glucose or insulin or induced glycemia and insulin response. Rivellese, et al, (1996, Diabetes Care 19(11):1207-13) showed that supplementation of subjects with impaired glucose control or Type 2 diabetes with 2-3 g of fish oil per day containing long-chain n-3 polyunsaturated fatty acid (PUFA) for 6 months did not alter serum insulin, fasting glucose, HbA1c levels or glucose tolerance tests. Stiefel et al., (1999, Ann Nutr Metab: 43(2):113-20) reported that administration of 330 mg DHA and 660 mg EPA per day resulted in a significant decrease in HbAlc levels in Type I diabetics. U.S. Patent 5,034,415 to Rubin (1991) reports a difference between naturally esterified fatty acids compared to the free fatty acid form in their effect on blood sugar levels. WO 02/11564 discusses nutritional supplements which may include lipid sources to be incorporated into the diet of diabetics. However, Friedberg, C.E. (1998 Diabetes Care 21(4):494-500) conducted a meta-analysis of 26 trials reported in the literature concerned with fish oil and diabetes. The analysis revealed that fish oil ingestion is associated with decrease in serum triglycerides and increase in LDL cholesterol, but without significant effect on HbA1c. Blood glucose showed borderline significant increases in Type II patients, which in the analysis appeared to be associated with DHA rather than EPA. Based on this meta-analysis of 26 trials, it would appear that fish oil could be useful for treating dyslipidemia in diabetics, but not for affecting glucose metabolism. Another recent meta-analysis of fish oil supplementation in T2DM by Montori et al., (2000 Diabetes Care: 23(9): 1407-1415) showed no statistically significant effect of fish oil on glycemic control as measured by fasting blood glucose or HbA1c. The triglyceride lowering effect of fish oil in T2DM was confirmed.

Studies with fish oil, which contains both EPA and DHA, clearly cannot differentiate among effects due to EPA, effects due to DHA, and effects that require both fatty acids. In a study by Shimura, et al. (1997 Biol. Pharm. Bull. 20(5):507-510) mice were dosed with DHA ethyl ester at 100 mg/kg body weight (e.g., 7 g/d for 70 kg man). This dose of DHA reduced blood glucose and plasma triglycerides and enhanced insulin sensitivity in obese diabetic mice, but not normal or lean diabetic mice. However, the KK-Ay mouse used by Shimura et al. is not reflective of the mechanism by which Type II diabetes develops in humans. The KK-Ay mouse is genetically obese and therefore develops Type II diabetes almost immediately after birth. In contrast, Type II diabetes in humans is obesity and age-related, typically developing after the age of 50 following at least a decade of impaired glucose tolerance and/or insulin insensitivity. A more appropriate mouse model, the NSY mouse, has become available. The NSY mouse develops Type II diabetes later in life following a disruption of the glucose/insulin metabolic response (Ueda et al., 2000, Diabetologia; 43(7):932-938). This more appropriate model has not been used in studies like those reported by Shimura, et al. In any case, the extremely high dose of fatty acid used in the Shimura study would be difficult and impractical for human therapy.

Research is also being done on the effect of omega-3 and omega-6 polyunsaturated fatty acids (PUFAs) on inflammation. Bockow disclosed in U.S. Patent 5,650,157 (July 22, 1997) a substantially natural form oil composition which includes omega-3 PUFAs for topical administration to reduce inflammation. Bockow et al. in U.S. Patent 5,411,988 (May 2, 1995) disclosed omega-3 and omega-6 PUFAs compositions which may include salicylate as site specific lavages for inflammation. U.S. application 2002/0055538 (May 9, 2002) discloses methods of treating inflammation using combinations of PUFAs which are hydroxylated in combination with aspirin. U.S. applications 2002/0137749 (September 26, 2002) and 2002/173510 (November 21, 2002) to Levinson et aL disclose various supplements for premenopausal and menopausal women which includes various PUFAs. U.S. application 2003/0064970 to Grainger et al. (April 3, 2003) discloses compounds and therapies or the prevention of vascular and non-vascular pathologies which include the use of omega PUFAs and aspirin. WO02/02 to Horrobin (January 10, 2002) discloses the preferred use of eicosapentaenoic acid (EPA) in combination with arachidonic acid (AA) to treat various conditions such as any psychiatric or neurological disease, asthma, gastrointestinal tract disorders, cardiovascular disease, diabetes and metabolic diseases. Similarly, U.S. application 2002/0169209 to Horrobin discloses the preferential administration of EPA with a COX-1, COX-2 or LOX inhibitor for many different disorders including cancers, skin disorders, inflammatory disorders, menstrual cycle disorders, metabolic disorders including diabetes mellitus, osteoporosis, urolithiasis and nervous systems disorders.

### SUMMARY OF INVENTION

It is an object of this invention to reduce subclinical inflammation in individuals. It is another object to reduce subclinical inflammation in individuals who are at risk for developing, or who currently have, atherosclerotic cardiovascular disease, coronary disease or cerebrovascular disease. It is another object to reduce subclinical inflammation in individuals at risk for developing, or who currently have, T2DM or who are prediabetic. It is another object of this invention to suppress or postpone development of macrovascular complications of diabetes by simultaneously enhancing glucose control and reducing the chronic subclinical inflammation associated with atherosclerotic disease, coronary disease or cerebrovascular disease.

These and other objectives are met by one or more of the following embodiments.

One embodiment provides methods and compositions for treating individuals exhibiting subclinical inflammation, preferably as assessed using inflammatory markers including CRP, vascular markers such as ICAM, VCAM and p-selectin, interleukins and cytokines, such as IL-1β, IL6, TNFα and LTB4. More preferably, the inflammatory marker used to assess subclinical inflammation is CRP. Another embodiment provides methods and compositions for treating subclinical inflammation associated with vascular related diseases.

Another embodiment provides compositions and methods for treating individuals at risk for developing T2DM.

In one embodiment, this invention provides methods which impede the development of coronary or cerebrovascular disease by prophylactic therapy for subclinical inflammation, especially in diabetic or prediabetic patients. In one embodiment, DHA is administered to the individual as a means of reducing C-reactive protein. In a particular embodiment, the method of this invention comprises administration of DHA substantially contemporaneously with an antiplatelet agents which is not ω-3 fatty acids; a particularly preferred antiplatelet agent is aspirin.

Therapy according to this invention is particularly preferred where the patient exhibits at least three symptoms selected from abdominal obesity, high triglycerides, low HDL cholesterol, high blood pressure and fasting glucose greater than about 100 mg/dL. More preferred patients are prediabetic or exhibit impaired glucose control, such as fasting glucose between about 110 to about 127 mg/dL or fasting insulin greater than about 6 µU/ml Particularly preferred are patients who exhibit triglycoride/HDL-C ratio of greater than 3.0 or exhibit blood HbAlc greater than about 7%. For such patients, successful application of therapy according to this invention means that onset of Type II diabetes mellitus is delayed, insulin sensitivity as measured by Frequently Sampled Intravenous Glucose Tolerance Testing (FSIGT) is improved, blood HbAlc is reduced in said patient, and/or the patient is protected against peripheral artery disease associated with both early type II and pre-type II diabetes.

In a clinical study in which DHA-containing single cell oil (DHASCO) capsules were co-administered with statin medication to patients with dyslipidemia, it was noted that HbAlc or glycosylated hemoglobin levels (a marker for glycemic control) were reduced in a clinically relevant manner in the high dose group (1000 mg DHA/day) after one year of treatment, when compared to the low dose group (200 mg DHA per day). Thus, the present inventors have discovered that DHA has a long term effect (as shown by reduction in glycosylated hemoglobin levels reflecting longer term glucose control integrated over 2-3 months). Finally, the inventors have discovered that therapy using DHA-containing oils can be effective at DHA levels that are not excessive (*e*.*g*., at levels which minimize side effects associated with fatty acid ingestion).

The same study indicated that, in a majority of subjects, DHASCO reduced levels of high specif city C-Reactive Protein (hs-CRP or CRP), a biomarker for chronic subclinical inflammation associated with increased CV risk, especially in persons with other CV risk factors such as low HDL cholesterol, insulin resistance and/or T2DM. In addition, the inventors have discovered that therapy using DHA-containing oils can be effective at DHA levels that are not excessive.

Thus, in another particular embodiment, this invention provides a method of treating patients with metabolic syndrome and/or an atherosclerotic disease and/or prediabetes by co-administering at least 1g/day of DHA as triglyceride oil, preferably with aspirin (ASA or acetylsalicylic acid) 35-325 mg/day, preferably 81 mg/day. Such chronic co-administration provides a novel approach to limiting the impact of several avenues to complications, morbidity and mortality from T2DM, prediabetes and/or an atherosclerotic disease and/or metabolic syndrome. The compositions and methods provide DHA which will improve glycemic control (as measured by HbAlc), lessening the metabolic derangements that predispose to vascular abnormalities that cause heart attacks and stroke. Additionally, the methods and compositions provide aspirin which will reduce platelet aggregation and hypercoagulability that, particularly in T2DM with vascular lesions, precipitates heart attack (coronary thrombosis) and/or stroke (cerebral thrombosis). The methods and compositions also provide the shared action of DHA and aspirin to reduce chronic subclinical inflammation that is strongly related to insulin resistance with its attendant atherosclerotic and clinical consequences described above.

In view of the discovery of (1) the effect ofDHA on glycosylation ofHb and (2) the effect of DHA with aspirin-mediated moderation of chronic subclinical inflammation (as measured by hs-CRP), such co-administration is useful, non-obvious and novel.

### BRIEF DESCRIPTION OF THE FIGURE

The Figure shows the average level of C-reactive protein (CRP) in patients before and after chronic administration of DHA.

### DETAILED DESCRIPTION OF THE EMBODIMENTS

Administration of DHA is effective in reducing levels of circulating C-reactive protein in patients that may be suffering from subclinical inflammation. These individuals may also be identified through the assessment of common risks factors such as those associated with stoke, including but not limited to increased age, hypertension, the presence of a carotid bruit, diabetes, smoking, atrial fibrillation, obesity, hyperlipidemia, and elevated homocysteine. Additional criteria may optionally include abdominal obesity (men >40" waist, women >35"), high triglycerides (≥150 mg/dL), low HDL cholesterol (men <40 mg/dL women <50 mg/dL), high blood pressure (≥130/≥85), plasma (or serum) CRP levels between about 3 mg/L and 10 mg/L, and high fasting glucose (>110 mg/dL). In particular the administration of DHA is particularly affective as a prophylactic treatment when an additional antiplatelet agent is included in the course of treatment.

Administration of DHA is effective in improving glycemic control in patients that may have metabolic syndrome with an increased risk of developing Type II diabetes. Metabolic syndrome is a constellation of lipid and non-lipid risk factors of metabolic origin. Metabolic syndrome is diagnosed when three or more of the following risk factors are present: abdominal obesity (men >40" waist, women >35"), high triglycerides (≥150 mg/dL), low HDL cholesterol (men <40 mg/dL women<50 mg/dL), high blood pressure (≥130/≥85), plasma (or serum) CRP levels between about 3 mg/L and 10 mg/L, and high fasting glucose (>110 mg/dL). Small LDL particle size is also characteristic of this syndrome. The estimated prevalence of metabolic syndrome in the US population is 24% and up to 42% for persons between 60 and 69 years of age. (Metabolic syndrome is also called "Syndrome X" or the "Insulin Resistance Syndrome [IRS]). JAMA 2001;285:2846-2897. Moderate to high doses (greater than 200 mg DHA per day) may provide improved glucose control, by a mechanism in which DHA lowers mean blood glucose.

### Target Patient Population

Patients who may benefit from therapy according to this invention include individuals who have been diagnosed as having an atherosclerotic disease, any of the above mentioned criteria or who have suffered from a stoke or TIA. This invention may also be used to treat patients with systemic low-grade inflammation, particularly patients with elevated C-reactive protein, an acute phase reactant associated with systemic and local inflammation (CRP), preferably with levels in excess of 3.9 mg/L (measured as described in Hafner, et al., Clin. Lab., 48:369-76 (2002)). Another criterion for suitable patients is elevated triglyceride/HDL-C ratio, especially a weight ratio of at least 4.6. This invention may also be used to treat hypertensive patients, recognizing that in addition to its demonstrated ability to reduce blood pressure (Mori et al., 1999 Hypertension. 34:253-260), as many as 50% of hypertensives go on to develop metabolic syndrome and/or type II diabetes. In a preferred aspect this invention treats individuals suffering from subclinical chronic inflammation, particularly individuals with a CRP level above about 3 mg/L, more preferably above about 5 mg/L in the absence of any acute inflammatory process. In another preferred aspect this invention treats individuals suffering from subclinical chronic inflammation associated with a vascular inflammatory disease. In another preferred aspect this invention treats individuals suffering from subclinical chronic inflammation associated with atherosclerosis.

Patients who may benefit from therapy according to the present invention include prediabetic patients, as well as, patients with overt diabetes. These may be patients with metabolic syndrome. In particular, it is preferred to treat patients with impaired glucose control as determined by a fasting glucose greater than 127 mg/dL, or even patients with fasting glucose greater than 110 mg/dL. An alternative criterion for suitable patients is fasting insulin greater than 6 gU/ml.

### Therapeutic Compositions

Suitable patients are treated according to this invention by chronic administration of a therapeutic composition containing DHA. Preferably the DHA. will be in the form of an oil for easier assimilation. (Triglycerides are a conventional source for dietary fatty acids.) More preferably, the oil will be substantially free of other ω-3 PUFA, in particular, no ω-3 PUFA other than DHA equal to 4% or more of the total fatty acid content. Even more preferably, the oil will be substantially free of EPA (*e*.*g*., <4% of Total Fatty Acid (TFA), or more preferably <3%, or more preferably <2%, and most preferably less than <1 %).

In one embodiment, DHA may be administered as a triglyceride oil containing at least 70% DHA, more preferably at least 75%, more preferably more than 80%, more preferably at least 85%, more preferably at least 90%, more preferably more than 95%, more preferably greater than 99%. To obtain a composition containing at least 70% of the fatty acids as DHA, one can subject a DHA-containing oil (e.g., a single cell oil from an algal source, such as Thraustochytriales or dinoflagellates) to hydrolysis and esterification to produce fatty acid monoesters, especially ethyl or methyl esters. The fatty acid esters are then subjected to known purification techniques, such as urea complexation, distillation, molecular distulation/fractionation, chromatography, etc., to recover a fraction with at least 70% DHA. The fractionated DHA mono esters, preferably C₁-C₄ alkyl chains, may be administered in that form, or the DHA may be transesterified to glycerol esters for administration or the esters may be hydrolyzed to provide free fatty acids for administration. C₁-C₄ alkyl groups may be either substituted (e.g. with hydroxyl, chloro, bromo, fluoro and iodo), unsubstituted, branched or unbranched. Non-limiting examples include methyl, ethyl, propyl, butyL

For each of the recited embodiments, the DHA may be administered from any number of sources and in varying amounts of purity. Preferably, the DHA is administered as an oil which substantially comprises DHA. In a more preferred embodiment, the DHA is a microbial oil with greater than 10% DHA, more preferably greater than 15% DHA, and more preferably greater than 20% DHA while preferably being substantially free of other PUPAs. In the above embodiments, DHA may be administered as a free fatty acid or ethyl ester thereof. Preferably, DHA is administered in a composition which contains no other PUFA, or which contains no other ω-3 PUPA greater than 4% of total fatty acid, or more preferably no greater than 3%, or more preferably no greater than 2% of total fatty acid, or more preferably no greater than 1% of total fatty acid, or administered in the absence of eicosapentaenoic acid (EPA). In another embodiment, DHA is administered in a composition which has an EPA content less than one-fifth that of DHA. In another embodiment, DHA is administered in a food product, which preferably contains less than one-fifth as much EPA as DHA. In another preferred embodiment, DHA, is administered in a triglyceride oil which contains no other long chain PUFA, which are meant to be PUFAs with C:20 or longer chains.

Although the DHA-containing oils can be administered to patients alone, more commonly, they will be combined with one or more pharmaceutically acceptable carriers and, optionally, other therapeutic ingredients. Acceptable carriers are those which are compatible with the other components of the formulation and not deleterious to the patient. It will be appreciated that the preferred formulation can vary with the condition and age of the patient.

The fatty acids may be from any source including, natural or synthetic oils, fats, waxes or combinations thereof Moreover, the fatty acids may be derived from non-hydrogenated oils, partially hydrogenated oils or combinations thereof Non-limiting exemplary sources of fatty acids include seed oil, fish or marine oil, canola oil, vegetable oil, safflower oil, sunflower oil, nasturtium seed oil, mustard seed oil, olive oil, sesame oil, soybean oil, com oil, peanut oil, cottonseed oil, rice bran oil, babassu nut oil, palm oil, low erucic rapeseed oil, palm kernel oil, lupin oil, coconut oil, flaxseed oil, evening primrose oil, jojoba, tallow, beef tallow, butter, chicken fat, lard, dairy butterfat, shea butter or combinations thereof. Specific non-limiting exemplary fish or marine oil sources include shellfish oil, tuna oil, mackerel oil, salmon oil, menhaden, anchovy, herring, trout, sardines or combinations thereof. Preferably, the source of the fatty acids is fish or marine oil, soybean oil or flaxseed oil, or microbially produced oil.

Particularly preferred oils are produced by microbial fermentation, as described in U.S. Patent Nos. 5,492,938 and 5,130,242, or International Patent Publication No. WO 94/28913, each of which is incorporated herein by reference in its entirety.

It is also possible for the dosage form to combine any forms of release known to persons of ordinary skill in the art. These include immediate release, extended release, pulse release, variable release, controlled release, timed release, sustained release, delayed release, long acting, and combinations thereof. The ability to obtain immediate release, extended release, pulse release, variable release, controlled release, timed release, sustained release, delayed release, long acting characteristics and combinations thereof is known in the art.

Any biologically acceptable dosage form known to persons of ordinary skill in the art, and combinations thereof, are contemplated. Examples of such dosage forms include, without limitation, chewable tablets, quick dissolve tablets, effervescent tablets, reconstitutable powders, elixirs, liquids, solutions, suspensions, emulsions, tablets, multi-layer tablets, bi-layor tablets, capsules, soft gelatin capsules, hard gelatin capsules, caplets, lozenges, chewable lozenges, beads, powders, granules, particles, microparticles, dispersible granules, cachets, douches, suppositories, creams, topicals, inhalants, aerosol inhalants, patches, particle inhalants, implants, depot implants, ingestibles, injectables (including subcutaneous, intramuscular, intravenous, and intradermal), infusions, health bars, confections, animal feeds, cereals, yogurts, cereal coatings, foods, nutritive foods, functional foods and combinations thereof. Most preferably the compositions and methods of the invention utilize a form suitable for oral administration.

Formulations of the present invention suitable for oral administration can be presented as discrete units, such as capsules or tablets, each of which contains a predetermined amount of DHA oil or a predetermined amount of a suitable combination of DHA oils. These oral formulations also can comprise a solution or a suspension in an aqueous liquid or a non-aqueous liquid The formulation can be an emulsion, such as an oil-in-water liquid emulsion or a water-in-oil liquid emulsion. The oils can be administered by adding the purified and sterilized liquids to a prepared enteral formula, which is then placed in the feeding tube of a patient who is unable to swallow.

Soft gel or soft gelatin capsules may be prepared, for example by dispersing the formulation in an appropriate vehicle (vegetable oils are commonly used) to form a high viscosity mixture. This mixture is then encapsulated with a gelatin based film using technology and machinery known to those in the soft gel industry. The industrial units so formed are then dried to constant weight.

In one preferred embodiment, the DHA microbial oil is incorporated into gel capsules. It will be recognized that any known means of producing gel capsules can be used in accordance with the present invention. Compressed tablets can be prepared by, for example, mixing the microbial oil(s) with dry inert ingredients such as carboxymethyl cellulose and compressing or molding in a suitable machine. The tablets optionally can be coated or scored and can be formulated so as to provide slow or controlled release of the pharmaceuticals therein. Other formulations include lozenges comprising DHA oil in a flavored base, usually sucrose and acacia or tragacanth.

Chewable tablets, for example may be prepared by mixing the formulation with excipients designed to form a relatively soft, flavored, tablet dosage form that is intended to be chewed rather than swallowed. Conventional tablet machinery and procedures, that is both direct compression and granulation, i.e., or slugging, before compression, can be utilized. Those individuals involved in pharmaceutical solid dosage form production are versed in the processes and the machinery used as the chewable dosage form is a very common dosage form in the pharmaceutical industry.

Film coated tablets, for example may be prepared by coating tablets using techniques such as rotating pan coating methods or air suspension methods to deposit a contiguous film layer on a tablet.

Compressed tablets, for example may be prepared by mixing the formulation with excipients intended to add binding qualities to disintegration qualities. The mixture is either directly compressed or granulated then compressed using methods and machinery known to those in the industry. The resultant compressed tablet dosage units are then packaged according to market need, i.e., unit dose, rolls, bulk bottles, blister packs, etc.

The invention also contemplates the use of biologically acceptable carriers which may be prepared from a wide range of materials. Without being limited thereto, such materials include diluents, binders and adhesives, lubricants, plasticizers, disintegrants, colorants, bulking substances, flavorings, sweeteners and miscellaneous materials such as buffers and adsorbents in order to prepare a particular medicated composition.

Binders may be selected from a wide range of materials such as hydroxypropylmethylcellulose, ethylcellulose, or other suitable cellulose derivatives, povidone, acrylic and methacrylic acid co-polymers, pharmaceutical glaze, gums, milk derivatives, such as whey, starches, and derivatives, as well as other conventional binders known to persons skilled in the art. Exemplary non-limiting solvents are water, ethanol, isopropyl alcohol, methylene chloride or mixtures and combinations thereof. Exemplary non-limiting bulking substances include sugar, lactose, gelatin, starch, and silicon dioxide.

The plasticizers used in the dissolution modifying system are preferably previously dissolved in an organic solvent and added in solution form. Preferred plasticizers may be selected from the group consisting of diethyl phthalate, diethyl sebacate, triethyl citrate, cronotic acid, propylene glycol, butyl phthalate, dibutyl sebacate, castor oil and mixtures thereof, without limitation. As is evident, the plasticizers may be hydrophobic as well as hydrophilic in nature. Water-insoluble hydrophobic substances, such as diethyl phthalate, diethyl sebacate and castor oil are used to delay the release of water-soluble vitamins, such as vitamin B6 and vitamin C. In contrast, hydrophilic plasticizers are used when water-insoluble vitamins are employed which aid in dissolving the encapsulated film, making channels in the surface, which aid in nutritional composition release.

Formulations suitable for topical administration to the skin can be presented as ointments, creams and gels comprising the DHA oil in a pharmaceutically acceptable carrier. A preferred topical delivery system is a transdermal patch containing the oil to be administered, In formulations suitable for nasal administration, the carrier is a liquid, such as those used in a conventional nasal spray or nasal drops.

Formulations suitable for parenteral administration include aqueous and non aqueous sterile injection solutions which optionally can contain antioxidants, buffers, bacteriostats and solutes which render the formulation isotonic with the blood of the intended recipient; and aqueous and non-aqueous sterile suspensions which can include suspending agents and thickening agents. The formulations can be presented in unit-dose or multi-dose containers. A preferred embodiment of the present invention includes incorporation of the DHA oil into a formulation for providing parenteral nutrition to a patient.

The microbial oil compositions of the present invention need not be administered as a pharmaceutical composition. They also can be formulated as a dietary supplement, such as a vitamin capsule or as food replacement in the normal diet. The microbial oils can be administered as a cooking oil replacement formulated so that in normal usage the patient would receive amounts of DHA sufficient to elevate the concentrations of this fatty acid in the serum and in membranes of affected patients. A special emulsion type margarine could also be formulated to replace butter or ordinary margarine in the diet. The single cell microbial oils could be added to processed foods to provide an improved source of DHA. The oil can be microencapsulated using gelatin, casein, or other suitable proteins using methods known in the art, thereby providing a dry ingredient form of the oil for food processing.

It should be understood that in addition to the ingredients particularly mentioned above, the formulations of this invention can include other suitable agents such as flavoring agents, preservatives and antioxidants. In particular, it is desirable to mix the microbial oils with an antioxidant to prevent oxidation of the DHA. Such antioxidants would be food acceptable and could include vitamin E, carotene, BHT or other antioxidants known to those of skill in the art.

### Therapeutic Protocols

In this invention DHA will be administered in an amount effective to reduce subclinical inflammation. The skilled clinician will monitor therapy and adjust doses as necessary. Markers of subclinical inflammation such as, CRP can be measured and dose adjusted to ensure that the marker of inflammation (as a surrogate for the condition) is reduced. Preferably in the case of CRP, reduced by a factor of 5%, more preferably 10%, more preferably 15%. Typically DHA will be administered in a high dose (greater than 200 mg/day), preferably at least 600 mg/day, more preferably greater than 800 mg/day, more preferably at least 1 g/day, more preferably greater than 1.1 g/day, more preferably greater than 1.2 g/day, more preferably greater than 1.3 g/day, more preferably greater than 1.4 g/day, or more preferably greater than 1.5 g/day while minimizing or eliminating side effects of excessive fatty acid dosing, such as belching, bloating, abdominal distress and other GI symptoms. In view of the side effects resulting from excess fatty acid administration, very high dose ω-3 fatty acid dosing is impractical as well as expensive, especially if fish oil is used as a source of DHA. Thus, the dose of DHA is preferably less than 7 g/day; more preferably less than 6 g/day; even more preferably less than 5 g/day. Amounts of DHA as described herein are expressed as the weight of DHA methyl ester equivalent to the DHA content of the dosage form. DHA may also be administered in conjunction with an anti-platelet agent, such as aspirin. DHA will be administered chronically, typically for at least 6 months, or at least one year, more preferably for two or more years, or for five or ten years or even for life.

For each of the recited embodiments of the invention, DHA administration is preferably chronic. In another embodiment, the DHA is administered in an amount greater than 200 mg/day, more preferably greater than 400 mg/day, more preferably greater than 600 mg/day, more preferably greater than 800 mg/day, more preferably greater than 1000 mg/day, more preferably greater than 1,100 mg/day, more preferably greater than 1,200 mg/day, more preferably greater than 1,500 mg/day. In another embodiment the amount of DHA is preferably less than 7 g/day, more preferably less than 6 g/day, more preferably less than 5 g/day, most preferably less than 4g/day. Intervening dosages, such as 300 mg/day, 400 mg/day, 500 mg/day, are also contemplated by the invention and the invention expressly contemplates any dosage greater than 200 mg/day, in 1 mg/day increments (e.g., 201 mg/day, 202 mg/day, 203 mg/day...301 mg/day, 302 mg/day, ...etc.).

Typically DHA will be administered to the patient in accordance with any embodiment of this invention on a periodic basis (*i,e*. chronically or episodically) in an amount greater than 200 mg/day, preferably at least 600 mg/day, more preferable at least 1000 mg DHA per day, even more preferably greater than 1.1 g DHA per day, while minimizing or eliminating side effects of excessive fatty acid dosing, such as belching, bloating, abdominal distress and other GI symptoms. In view of the side effects resulting from excess fatty acid administration, very high dose ω-3 fatty acid dosing is impractical as well as expensive, especially if fish oil is used as a source of DHA. Thus, the dose of DHA is preferably less than 7 g/day; more preferably less than 6 g/day; even more preferably less than 5 g/day. DHA will typically be administered periodic basis, such as for at least 3 months, 6 months, or at least one year, more preferably for two or more years, or for five or ten years or even for life. The DHA may also be administered as a triglyceride oil, preferably containing at least 70% DHA, or a triglyceride oil which contains no other ω-3 PUFA greater than 2% of total fatty acid. Preferably the DHA is administered in the absence of eicosapentaenoic acid (BPA) or in a triglyceride oil which has an EPA content less than one-fifth that of DHA, preferably in a food product that contains less than one-flfth as much EPA as DHA.

Compositions of the invention may be administered in a partial, i.e., fractional dose, one or more times during a 24 hour period, a single dose during a 24 hour period of time, a double dose during a 24 hour period of time, or more than a double dose during a 24 hour period of time. Fractional, double or other multiple doses may be taken simultaneously or at different times during the 24 hour period. The doses may be uneven doses with regard to one another or with regard to the individual components at different administration times.

One suitable therapeutic regimen would be to administer approximately 1000 mg of DHA as DHASCO (i.e., DHA-containmg single cell oil) capsules to patients with elevated levels of C-reactive protein. The patients would continue to take DHA chronically with the goal of delaying the onset of cardiovascular disease, cardiovascular disease related to metabolic syndrome or reducing clinical inflammation associated with atherosclerotic disease.

In accordance with this invention, administration of DHA as described herein will delay onset of an atherosclerotic disease or assist in alleviating associated symptoms. Therapy according to this invention may also delay onset of metabolic syndrome. For the purposes of this invention protection against a disease or disease state such as coronary artery disease, cerebrovascular disease or peripheral artery disease is meant to include a reduction in the risk for the disease, a delay in disease onset, or a need for a reduced medical routine including doctor visits and/or medication dosages or frequency. Further, protection against a disease also includes the prevention or amelioration of at least one symptom associated with the disease or disease state. Effectiveness of therapy according to this invention may also be detected by intermediate measurement of improved insulin sensitivity (as measured by, e.g., FSIGT), or improved glucose control detected by reduced blood HbAlc at or below 7%. Therapy according to this invention may also protect against peripheral artery disease in both early type II or pre-type II diabetes.

The dose of DHA for a particular patient can be determined by the skilled clinician using standard pharmacological approaches in view of the above factors, The response to treatment may be monitored by analysis of blood or body fluids in the patient The skilled clinician will adjust the dose and duration of therapy based on the response to treatment revealed by these measurements.

### Combination Therapy

DHA may be used alone, but in particularly preferred embodiments, it is administered concurrently with one or more other therapeutic agents. The concurrent agents may be directed at the same symptomatic or causative effects, or at different therapeutic targets. "Concurrent administration of two agents" as used herein means that both agents are present in pharmacologically effective levels in the circulation at the same time. Concurrent administration may be achieved by formulating both agents in the same composition, but it may also be achieved by simultaneous ingestion of doses of each agent or by administration of the two agents sequentially, so long as pharmacological effectiveness is achieved. Combination packaging described below with indicia for concurrent administration is contemplated by this invention.

Substantially contemporaneously means delivery of a second pharmaceutical, preferably an antiplatelet and/or an antidiabetic, within twenty-four hours of delivery of a DHA, dosage of the invention. More preferably the second pharmaceutical is delivered within 12 hours, more preferably 6 hours, and more preferably 1 hour of delivery of the second pharmaceutical. In another embodiment, it is preferred that a DHA dosage is provided within 1 hour of delivery of the second pharmaceutical, more preferably 45 minutes, more preferably 30 minutes, and most preferably within 15 minutes of delivery of the second pharmaceutical.

Likewise, the compositions of the invention may be provided in a blister pack or other such pharmaceutical package. Further, the compositions of the present inventive subject matter may further include or be accompanied by indicia allowing individuals to identify the compositions as products for inflammation and/or glucose regulation. The indicia may further additionally include an indication of the above specified time periods for administering the compositions. For example the indicia may be time indicia indicating a specific or general time of day for administration of the composition, or the indicia may be a day indicia indicating a day of the week for administration of the composition. The blister pack or other combination package may also include a second pharmaceutical product, e.g. a typical antiplatelet medication, which should be taken in addition to the compositions of the invention. It should be understood from this disclosure that the second pharmaceutical may be an antiplatelet agent. In a separate embodiment there may be additional agents delivered such as an anti-diabetic medication which is known in the art, as many individuals suffering from diabetes are also at risk for atherosclerotic diseases. Particularly preferred are combination packages with at least two of the above three agents.

In a particular embodiment, this invention provides a method for treating an individual diagnosed with an atherosclerotic disease, a hypertensive disease and/or prediabetic patients by concurrent administering of more than 200 mg/day, preferably at least 1 g/day of DHA, preferably as triglyceride oil, and an anti-platelet agent, such as aspirin (typically 81-325 mg) to patients with metabolic syndrome and/or impaired glucose control (but not yet necessarily diagnosed with Type II diabetes) as measured by elevated fasting glucose levels (110-127 mg/dl) and/or elevated fasting insulin levels (>6 µU/ml) and essential hypertension (blood pressure equal to or greater than 140/90 mmHg). Concurrent administration of DHA with aspirin or with other anti-platelet agents will reduce platelet aggregation and hypercoagulability which, especially in Type II diabetes patients, lead to vascular lesions associated with coronary heart disease and thrombosis associated with stroke.

Another suitable therapeutic regimen would be to administer approximately 1000 mg of DHA as DHASCO (i.e., DHA-containing single cell oil) capsules with an anti-platelet, most preferably aspirin, to patients with elevated levels of C-reactive protein. The patients would continue to take DHA chronically and with the second pharmaceutical with the goal of delaying the onset of cardiovascular disease, cardiovascular disease related to metabolic syndrome or reducing inflammation associated with vascular diseases, such as atherosclerotic disease,

Antiplatelet drugs protect against myocardial infarction, stroke, cardiovascular death and other serious vascular events in patients with a history of previous vascular events or known risk factors for cardiovascular disease. The major role of antiplatelet drugs in clinical practice is to prevent the adverse clinical sequelae of thrombosis in atherosclerotic arteries to the heart (acute coronary syndromes (ACS), brain (ischemic stroke), and limbs (intermittent claudication and rest pain); and thrombosis of stagnant blood in veins (venous thromboembolism) and heart chambers (atrial fibrillation, heart failure). Aspirin reduces the risk of serious vascular events in patients at high risk of such an event by about a quarter and is recommended as the first-line antiplatelet drug. Aspirin, clopidogrel, dipyridamole and the glycoprotein IIb/IIIa receptor antagonists (abciximab and tirofiban) are examples of antiplatelet drugs.

Aspirin (acetylsalicylic acid) irreversibly inhibits prostaglandin H synthase (cyclooxygenase-1) in platelets and megakaryocytes, and thereby blocks the formation of thromboxane A2 (TXA2; a potent vasoconstrictor and platelet aggregant). It is only the parent form, acetylsalicylic acid, which has any significant effect on platelet function. Evidence indicates daily doses of aspirin in the range 75-150 mg for the long-term prevention of serious vascular events in high risk patients is as effective as higher doses of 500-1500 mg aspirin daily. Higher doses are typically given for clinical inflammation, for instance, people with arthritis may take as much as 4,000 mg of aspirin every day. However, aspirin use at higher levels is associated with dose-related symptoms of upper-GI toxicity (nausea, heart bum, epigastric pain). Thus, antiplatelet therapy according to this invention contemplates aspirin below 500 mg/day.

The thienopyridine derivatives (clopidogrel and ticlopidine) are metabolised in the liver to active compounds which covalently bind to the adenosine phosphate (ADP) receptor on platelets and dramatically reduce platelet activation. Clopidogrel reduces the risk of serious vascular events among high-risk patients by about 10% compared with aspirin. It is as safe as aspirin, but much more expensive. It is an appropriate alternative to aspirin for long-term secondary prevention in patients who cannot tolerate aspirin, have experienced a recurrent vascular event while taking aspirin, or are at very high risk of a vascular event (≥20% per year).

An oral loading dose of 300-600 mg clopidogrel produces detectable inhibition of ADP-induced platelet aggregation after 2 hours, which becomes maximal after 6 hours. If a loading dose of clopidogrel is not used, repeated daily oral doses of 75 mg clopidogrel are required to achieve a steady-state maximal platelet inhibition, which is comparable with that produced by 250 mg ticlopidine orally, twice daily. Compared with aspirin, the thienopyridines are associated with a lower risk of GI hemorrhage and upper-GI symptoms and an increased risk of diarrhea and of skin rash. Ticlopidine doubles the risk of skin rash and diarrhea compared with aspirin, whereas clopidogrel increases skin rash and diarrhea by about a third, compared with aspirin.

Dipyridamole inhibits phosphodiesterase, which inactivates cyclic AMP increases intraplatelet concentrations of cyclic AMP and reduces the activation of cytoplasmic second messengers. Dipyridarnole also stimulates prostacyclin release and inhibits thromboxane A2 formation. Because the effect is short-lasting, repeated dosing or slow-release preparations are required to inhibit platelet function for 24 hours.

Glycoprotein IIb/IIIa receptor antagonists block the final common pathway for platelet aggregation. Abciximab is a humanized mouse antibody fragment with a high binding affinity for the glycoprotein IIb/IIIa receptor. Tirofiban (a non-peptide derivative of tyrosine) and eptifibatide (a synthetic heptapeptide) mimic part of the structure of fibrinogen that interacts with the glycoprotein IIb/IIIa receptor and thus compete with ligand binding of fibrinogen to the glycoprotein IIb//IIIa receptor. Glycoprotein IIb/IIIa receptor antagonists are given intravenously as a bolus injection, followed by a continuous infusion for up to 72 hours. At 24 hours after cessation of an infusion of abciximab, there is persistent blockade of more than 50% of platelet glycoprotein IIb/IIIa receptors, but platelet function recovers after 2 days. By contrast, the antiplatelet effects of tirofiban rapidly dissipate after cessation of the infusion.

Typically, antiplatelet agents according to any recited embodiment of this invention will be administered to the patient on a periodic basis (i.e. chronically or episodically) in an amount equivalent to aspirin between 35 mg/day and 400 mg/day, more preferably between 35 mg/day and 375 mg/day, more preferably between 35 mg/day and 350 mg/day, more preferably between 35 mg/day and 325 mg/day, more preferably between 35 mg/day and 300 mg/da.y, more preferably between 35 mg/day and 275 mg/day, more preferably between 35 mg/day and 250 mg/day, more preferably between 35 mg/day and 225 mg/day, more preferably between 35 mg/day and 200 mg/day, more preferably between 35 mg/day and 175 mg/day, more preferably between 35 mg/day and 150 mg/day, more preferably between 35 mg/day and 125 mg/day, more preferably between 35 mg/day and 100 mg/day, more preferably between 50 mg/day and 100 mg/day, more preferably between 75 mg/day and 100 mg/day, and most preferably about 81 mg/day.

Research has shown that aspirin dosages between 75-150 mg/day decrease the incidence of coronary heart disease in adults who are at increased risk and individuals at increased cardiovascular risk who may wish to consider long-term aspirin therapy. Risk groups typically include men older than 40 years of age, postmenopausal women, and younger people with risk factors for cardiovascular disease. Risk factors for cardiovascular disease include increasing age, male sex, cigarette smoking, increasing blood pressure, increasing blood total cholesterol concentration, decreasing high-density lipoprotein cholesterol concentration, raised fasting blood glucose concentration (i.e., diabetes mellitus), and a positive family history of cardiovascular disease (in younger adults).

Compositions and methods of the invention may provide a reduction in the risk factors for hemorrhagic complications of aspirin and other antiplatelet drugs including severe or continuing diarrhea, heavy or unusual menstrual bleeding, continued bleeding due to falls, injuries, or blows to the body or head, bleeding gums, unusual bruises or purplish areas on the skin, and unexplained nosebleeds.

### Examples

In order to facilitate a more complete understanding of the invention, Examples are provided below. However, the scope of the invention is not limited to specific embodiments disclosed in these Examples, which are for purposes of illustration only.

### Example 1

In a clinical study DHASCO capsules (which contained DHA as a triglyceride oil extracted from *Crypthecodinium cohnii* cells, obtained from Martek Biosciences Corp., Columbia, Maryland) were co-administered with statin medication to patients with dyslipidemia. Hyperlipidemic patients already being treated with a stable dose of a statin medication but still failing to meet NCEP guidelines for LDL-cholesterol or triglycerides were treated with either 200 or 1000 mg of DHA daily for 12 months. HbAlc levels (glycosylated hemoglobin, a marker of glycemic control) were measured in plasma at baseline and after 8 or 12 months of treatment. The HBA1c levels were significantly reduced in the high dose group (1000 mg DHA/day) after one year of treatment compared to the low dose group (200 mg DHA per day).

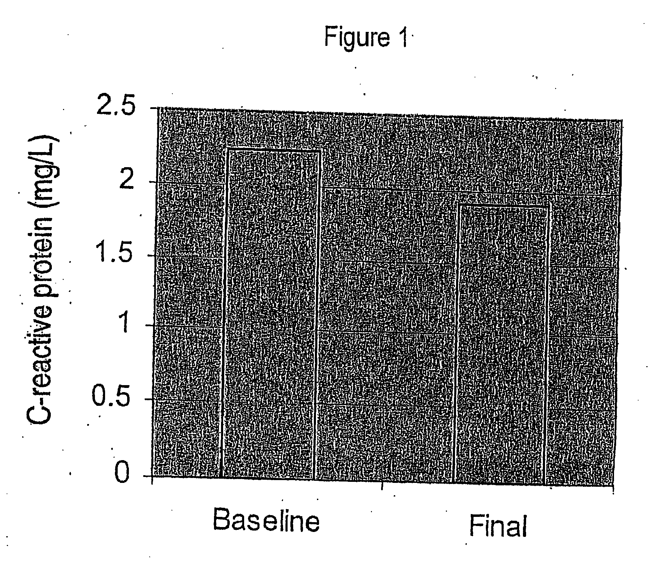

In this study, thirteen of 20 patients treated with DHA showed reductions in CRP levels, for an overall reduction of 15% in CRP level. Reduction in CRP of this extent is clinically significant, and may be correlated with a benefit of reduced risk of Type II diabetes onset, independent of other Type II risk factors. These results are shown in Figure 1.

### Example 2

To validate the results of the clinical trial described in Example 1, a population of 300 individuals who have suffered a heart attack may be selected for study. All members of the study will be tested for C-reactive protein levels and the inflammatory markers IL-6, ICAM, VCAM, p-selectin, TNFα, LTB4 and for peripheral blood mononuclear cell immune reactivity (PBMC, e.g. white blood cells). Alternatively, at least three of the above markers may be selected for monitoring in the study. The population will then be randomly divided into two treatment groups. The first treatment group will receive DHA according to the invention in the amount of 1 g/day in capsules containing a triglyceride oil that is 50% DHA. The second treatment group will receive a placebo which will contain soybean oil or a suitable substitute in the amount of 2 g/day. Each group will maintain the treatment course for a period of at least six months to a year. Over the evaluation period info marker testing will be assessed monthly. Additionally, at least at the onset and conclusion of the study individuals will be assessed for their HbAlc levels. Upon completion of the DHA supplementation study, the DHA group may be expected to show a reduction in the mean C-reactive protein concentration and moderated levels of other inflammatory markers compared to baseline and compared to the placebo group. Each group will also be monitored for cardiovascular events, including myocardial infarct, stroke TIA, exacerbation of peripheral vascular attack, or related acute event.

### Example 3

To validate the effectiveness of the combination therapy, a clinical trial a population of 300 individuals who have suffered a heart attack may be selected for study. All members of the study will be tested for C-reactive protein levels and the inflammatory markers IL-6, ICAM, VCAM, p-selectin, TNFα; LTB4 as well as PBMC immune reactivity. Alternatively, at least three of the above markers may be selected for monitoring during the study. The population will then be randomly divided into two treatment groups. The first treatment group will receive DHA in the amount of 1 g/day and 81 mg/day of aspirin. The second treatment group will receive a placebo which will contain soybean oil or a suitable substitute in the same amount based on TFA and 81 mg/ml of aspirin. Each group will maintain the treatment course for a period of at least six months to a year. Over the evaluation period inflammatory marker testing will be performed monthly Additionally, at least at the onset and conclusion of the study individuals will be assessed for their HbAlc levels. Each group will also be monitored for cardiovascular events, including myocardial infarct, stroke, TIA, exacerbation of peripheral vascular attack, or related acute event. Upon completion, the DHA/aspirin group may be expected to have a reduction in mean CRP and moderated levels of other inflammatory marker as compared to baseline and as compared to the placebo group.

Although the foregoing invention has been described in some detail by way of illustration and example for purposes of clarity of understanding, it will be obvious that certain changes and modifications may be practiced within the scope of the appended claims. Modifications of the above-described modes for carrying out the invention that are obvious to persons of skill in medicine, pharmacology, and/or related fields are intended to be within the scope of the following claims.

All publications and patent applications mentioned in this specification are indicative of the level of skill of those skilled in the art to which this invention pertains. All such publications and patent applications are herein incorporated by reference in their entirety to the same extent as if each individual publication or patent application was specifically and individually indicated to be incorporated by reference in their entirety.
1. A method for impeding the development or progression of a disease associated with subclinical inflammation comprising administering docosahexaenoic acid (DHA) to a patient in an amount effective to reduce subclinical inflammation.
2. The method of clause 1, wherein said disease is cerebrovascular disease, coronary artery disease or peripheral artery disease.
3. The method of clause 1, wherein said patient is suffering from type 2 diabetes mellitis (T2DM), metabolic syndrome or hypertension.
4. A method of prophylactic therapy for subclinical inflammation comprising administering DHA to a patient having an elevated level of circulating CRP, wherein said DHA is administered in an amount sufficient to reduce circulating CRP in the patient.
5. The method according to any one of clauses 1,2, 3, or 4 comprising administering an effective amount of DHA substantially contemporaneous with a second medicament to a patient, wherein said DHA and said second medicament are administered in an amount sufficient to reduce circulating C reactive protein in the patient.
6. The method according to clause 5, wherein said second medicament is an antplatelet agent.
7. The method of clause 6, wherein the antiplatelet agent is aspirin, clopidogrel, a glycoprotein IIb/IIIa receptor antagonist, or combinations thereof.
8. The method of clause 7, wherein the antiplatelet agent is aspirin.
9. The method of clause 8, wherein from 35-250 mg aspirin is administered per day.
10. The method of clause 1,2, 3,4, 5, or 6 wherein the patient is a diabetic.
11. The method of clause 1,2, 3,4, 5, or 6 wherein the patient is a prediabetic.
12. The method of clause 1,2, 3,4, 5, or 6 wherein said patient is protected against peripheral artery disease associated with both early type II and pre-type II diabetes.
13. The method of any preceding clause wherein the patient exhibits at least three symptoms selected from abdominal obesity, high triglycerides, low HDL cholesterol, high blood pressure and fasting glucose greater than 100 mg/dL.
14. A method of treating an individual at risk of having a stroke comprising: a) assessing an individual to determine if three or more risk factors are present wherein the risk factors are selected from abdominal obesity (men > 40"waist, women > 35"), high triglycerides (50 mg/dL), low HDL cholesterol (men < 40 mg/dL women < 50 mg/dL), high blood pressure (> 30/35), small LDL particle size and high fasting glucose (> 110 mg/dL) in combination with elevated levels of C-reactive protein; b) providing said individual with a dosage of DHA which is greater than about 750 mg/day for a period of more than three months.
15. The method of clause 14, wherein the individual is also administered aspirin.
16. The method of any preceding clause wherein said administration of DHA is chronic.
17. The method of any preceding clause wherein DHA makes up at least about 70% of the fatty acids administered as a triglyceride oil, free fatty acids, fatty acid alkyl esters or combinations thereof.
18. The method of any preceding clause wherein DHA is administered in a triglyceride oil which contains no other-3 PUFA greater than about 4% of total fatty acid.
19. The method of any preceding clause wherein DHA is administered in a triglyceride oil which has an EPA content less than about one-fifth that of DHA.
20. The method of any preceding clause wherein DHA is administered in a food product that contains DHA as a triglyceride oil, free fatty acids, fatty acid alkyl esters or combinations thereof.
21. The method of clause 8 wherein 200 mg/day to 500 mg/day of DHA are administered and wherein 81 mg/day to 162 mg/day of aspirin are administered.

## Claims

1. Use of DHA in in the preparation of a first medicament for reducing the risk of an individual developing a disease selected from coronary artery disease, coronary disease and cerbrovascular disease comprising administering an oil or formulation to an individual who is at risk of developing the disease and who is assessed as having an elevated CRP level wherein the oil or formulation comprises DHA and is substantially free of another polyunsaturated acid and wherein the DHA is administered to the individual in an amount of from 200 mg/day to 4g/day.

2. Use according to claim 1 wherein the individual is further assessed as having a risk factor selected from a from the group consisting of increased age, hypertension, the presence of a carotid bruit, diabetes, smoking, atrial fibrillation, obesity, hyperlipidemia, elevated homocystein, abdominal obesity, high triglycerides, low HDL cholesterol, high blood pressure, and high fasting glucose.

3. Use according to any preceding claim wherein the oil or formulation comprises >20% DHA of total fatty acid.

4. Use according to any preceding claim where the DHA is a triglyceride.

5. Use according to any preceding claim wherein the DHA is an ethyl ester.

6. Use according to any preceding claims wherein the oil or formulation is substantially free of EPA.

7. Use according to any preceding claim wherein the oil or formulation comprises >70% DHA of total fatty acid.

8. Use according to any preceding claim wherein the DHA is administered 1 gram/day.
